# EUROPEAN PATENT SPECIFICATION

(11) **EP 3 190 893 B1**
(45) Date of publication and mention of the grant of the patent: **12.05.2021**
(21) Application number: 15839779.4
(22) Date of filing: 11.09.2015
(51) Int. Cl.: A01N 59/00, A61K 33/00, A61P 31/04

(54) **COMPOSITIONS AND METHODS FOR TREATING AND PREVENTING BACTERIAL INFECTIONS**
ZUSAMMENSETZUNGEN UND VERFAHREN ZUR BEHANDLUNG UND VORBEUGUNG VON BAKTERIELLEN INFEKTIONEN
COMPOSITIONS ET PROCÉDÉS DE TRAITEMENT ET DE PRÉVENTION D'INFECTIONS BACTÉRIENNES

(30) Priority: 12.09.2014 US 201462049915 P
(43) Date of publication of application: 19.07.2017
(73) Proprietor: K10 Technologies, Inc., Santa Monica, California 90403 (US)
(72) Inventor: MATLICK, John, Santa Monica, California 90403 (US)
(74) Representative: CSY London
(86) International application number: PCT/US2015/049657
(87) International publication number: WO 2016/040785

(56) References cited:
- WO-A1-2008/095276
- WO-A1-2014/015444
- WO-A2-2013/166614
- JP-A- H10 225 507
- US-A1- 2002 187 203
- US-A1- 2004 110 738
- US-A1- 2008 050 452
- US-A1- 2009 110 750
- US-A1- 2010 003 330
- US-A1- 2010 183 739
- US-A1- 2012 219 638
- US-A1- 2013 171 228
- LO, SY ET AL.: 'Evidence for the existence of stable-water-clusters at room temperature and normal pressure.' PHYSICS LETTERS A. 12 October 2009, XP026623932

## Description

### CROSS REFERENCE TO RELATED APPLICATIONS

This application claims priority under 35 U.S.C. §119(e) from U.S. Provisional Application No. 62/049,915 filed on September 12, 2014.

### FIELD OF THE INVENTION

This invention generally relates to compositions for treating, preventing, lessening the severity of and promoting the prophylaxis of bacterial infections

### BACKGROUND

The following description includes information that may be useful in understanding the present invention. It is not an admission that any of the information provided herein is prior art or relevant to the presently claimed invention, or that any publication specifically or implicitly referenced is prior art.

Widespread use of traditional antibiotics is responsible for the emergence and rapid spread of resistant pathogens, such as methicillin-resistant *S. aureus* (MRSA), or *S. Pneumoniae,* which highlights a pressing need for development of novel antimicrobial therapies.

### SUMMARY OF THE INVENTION

In various embodiments, the invention teaches a composition according to claim 1 for use in treating a bacterial infection in a subject. In some embodiments, the bacterial infection is a bacterial infection caused by a Gram-negative bacterium. In some embodiments, the bacterial infection is a bacterial infection caused by a Gram-positive bacterium. In certain embodiments, the bacterial infection is a bacterial infection caused by a bacterium selected from the group consisting of *S. pyogenes, S. Mutans, S. pneumonia, S. aureus, K. pneumoniae* and combinations thereof. In certain embodiments, the bacterial infection is a bacterial infection on a portion of the subject's body selected from the group consisting of: an interior surface of
a subject's body, an external surface of a subject's body, and a mucous membrane of a subject's body. In some embodiments, the subject is a mammal. In some embodiments, the subject is a human. In certain embodiments, the composition is administered in a form selected from the group consisting of a powder, a liquid, a solid, a nebulized particle, an atomized particle, and combinations thereof.

In various aspects, the disclosure teaches a method for treating a bacterial infection in a plant, including administering an effective amount of a composition including a water cluster to the plant and/or the soil in which it resides. In certain aspects the plant is an edible crop. In certain aspects, the plant is not edible. In some aspects, the composition is administered in a form selected from the group consisting of a powder, a liquid, a solid, a nebulized particle, an atomized particle, and combinations thereof.

In various aspects, the disclosure teaches a method for preventing or slowing the growth of a bacterium in a food, including applying an effective amount of a composition including a water cluster to the food. In certain aspects, the food is suitable for consumption by an animal. In some aspects, the food is suitable for consumption by a human. In certain aspects, the composition is applied to the food in a form selected from the group consisting of a powder, a liquid, a solid, a nebulized particle, an atomized particle, and combinations thereof.

In various aspects, the disclosure teaches a method for slowing or preventing the growth of a bacterium on a non-living substrate, including applying an effective amount of a composition including a water cluster to the non-living substrate. In some aspects, the bacterium is selected from the group consisting of *S*. *pyogenes, S. Mutans, S. pneumonia, S. aureus* and combinations thereof.

### BRIEF DESCRIPTION OF THE DRAWINGS

Exemplary embodiments are illustrated in referenced figures. It is intended that the embodiments and figures disclosed herein are to be considered illustrative rather than restrictive.

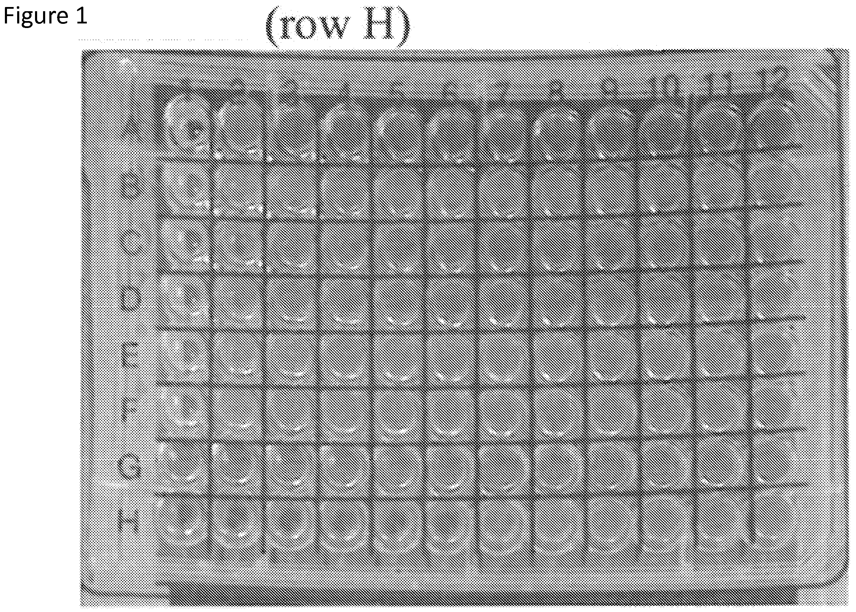

Figure 1 depicts, in accordance with an embodiment of the invention, *Streptococcus pneumoniae* (*S. pneumonia*) ATCC 49619 treated with (1) water clusters suspended in water (at approximately 150 million water clusters per drop of water with an average water droplet size of 500 microns in diameter) before dehydration (rows A-B); after dehydration of 2 mL in glass and subsequent rehydration with 200 µL of sterile water (rows C-D); after dehydration of 2 mL in plastic and subsequent rehydration with 200 µL of sterile water (rows E-F); and (2) Linezolid (row H).

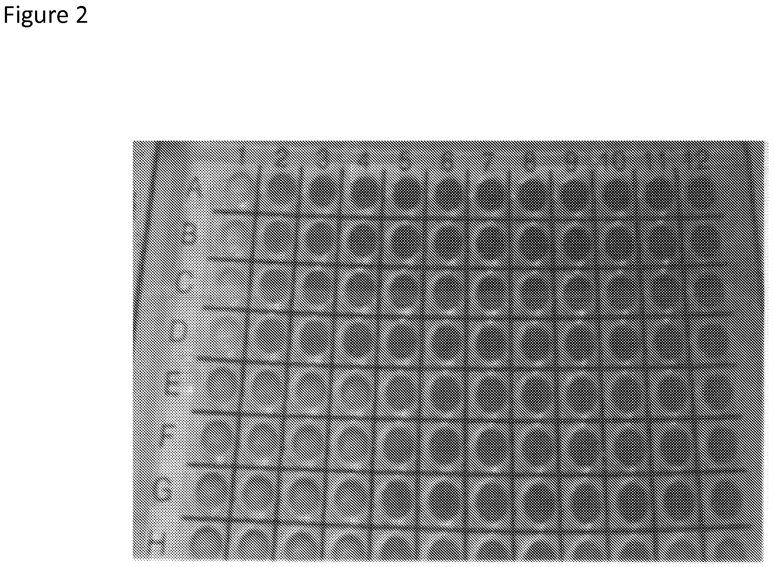

Figure 2 depicts, in accordance with an embodiment of the invention, *S. pneumoniae* ATCC 49619 treated with (1) water clusters suspended in water (at approximately 150 million water clusters per drop of water with an average water droplet size of 500 microns in diameter). (rows A-D); and (2) linezolid (rows E-H).

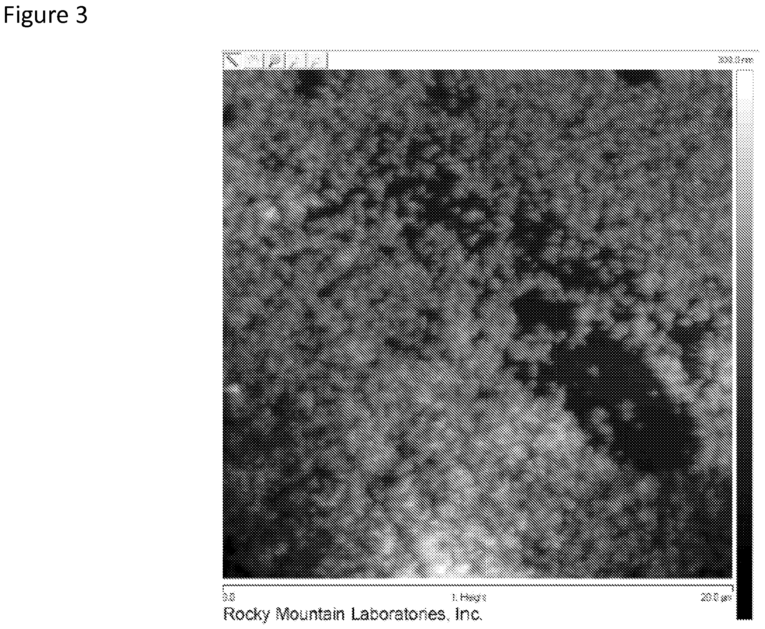

Figure 3 depicts, in accordance with an embodiment of the invention, a 20µM x 20µM atomic force microscopy (AFM) image of water clusters remaining after ultrapure water containing water clusters (at the concentrations described in figures 1 and 2) is evaporated.

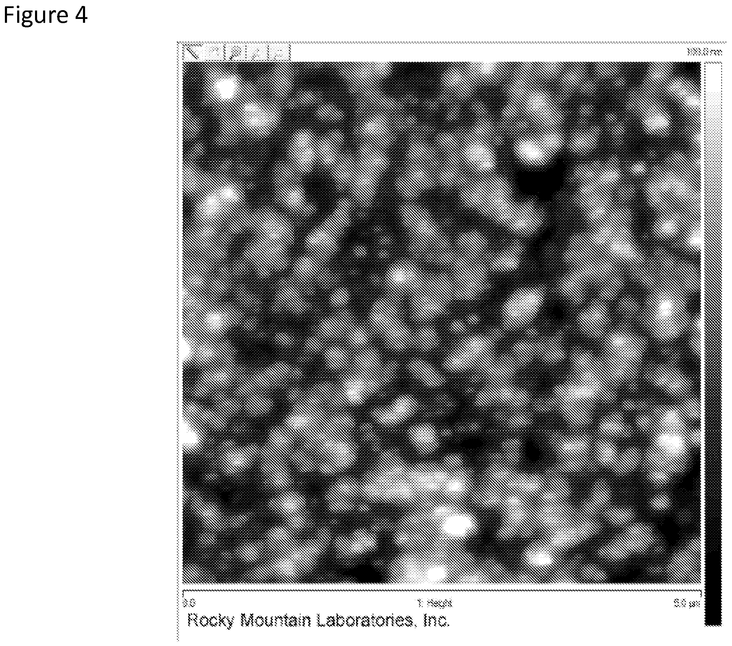

Figure 4 depicts, in accordance with an embodiment of the invention, a 5µM x 5µM AFM image of water clusters remaining after ultrapure water containing water clusters (at the concentrations described in figures 1 and 2) is evaporated.

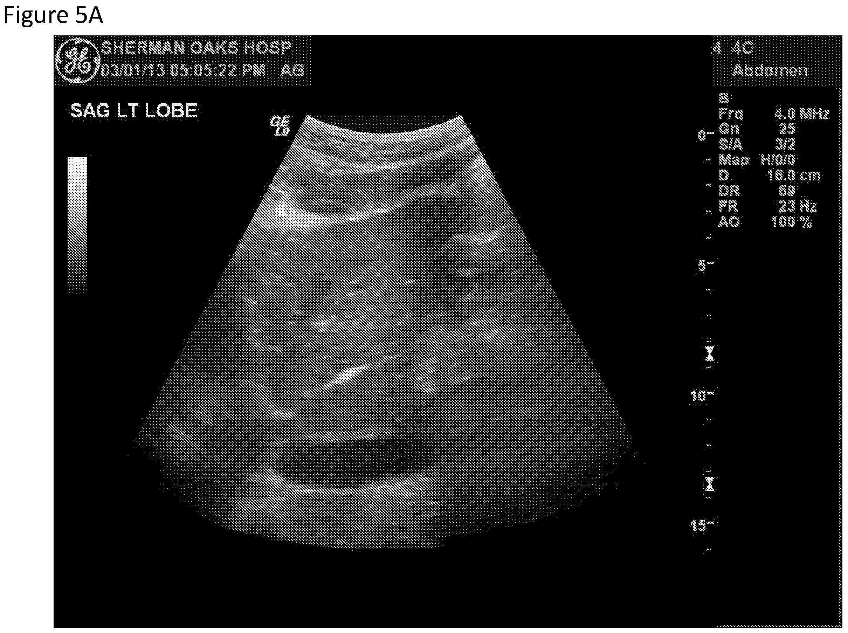

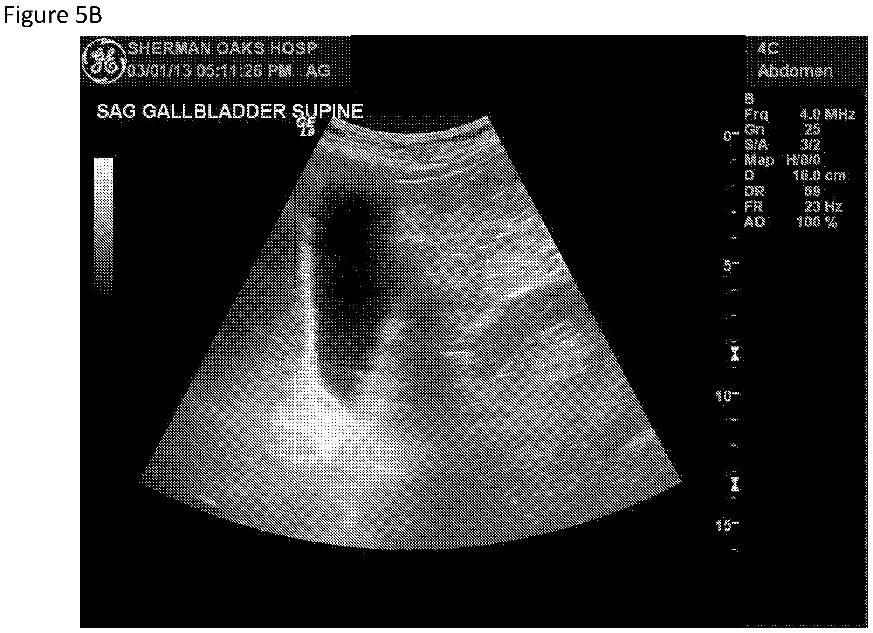

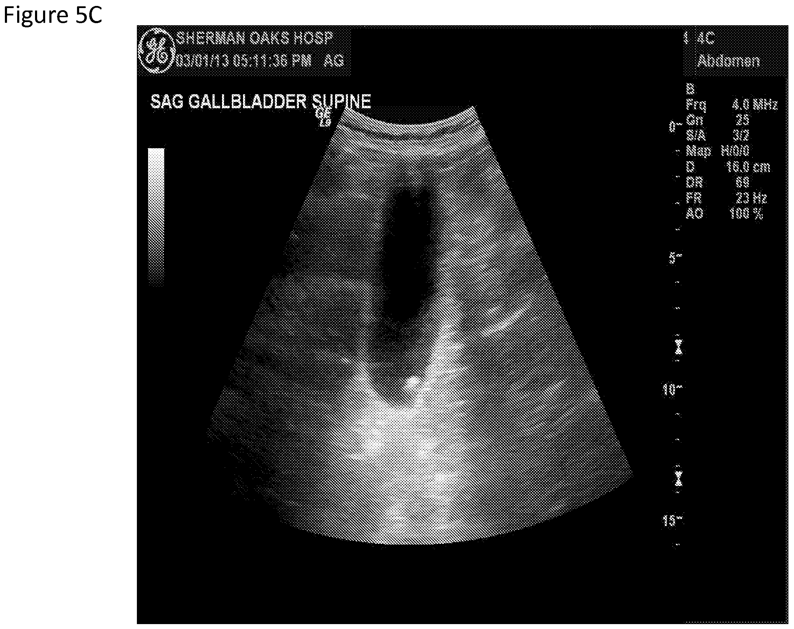

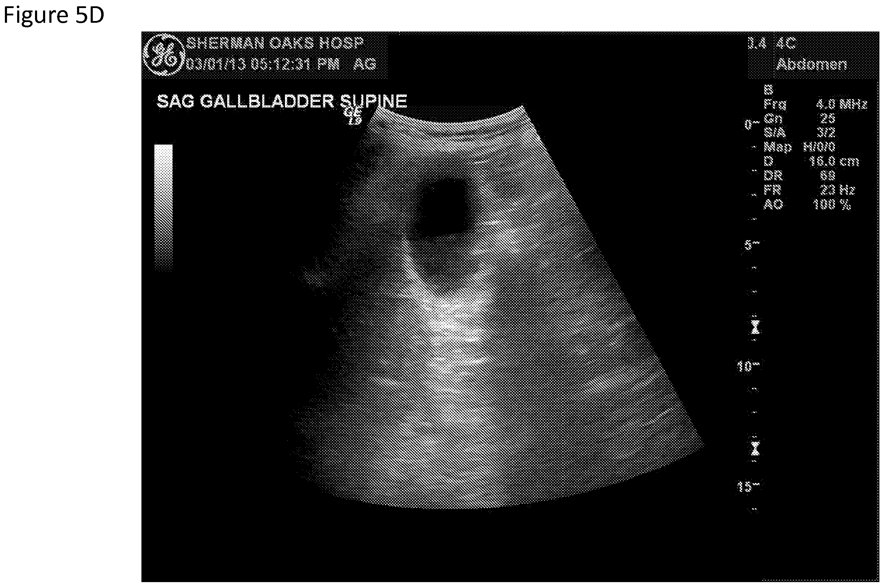

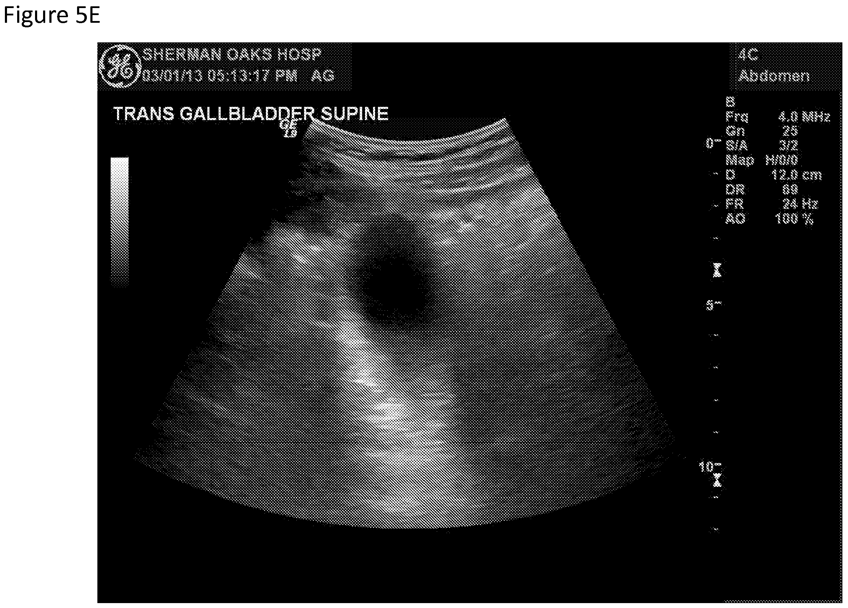

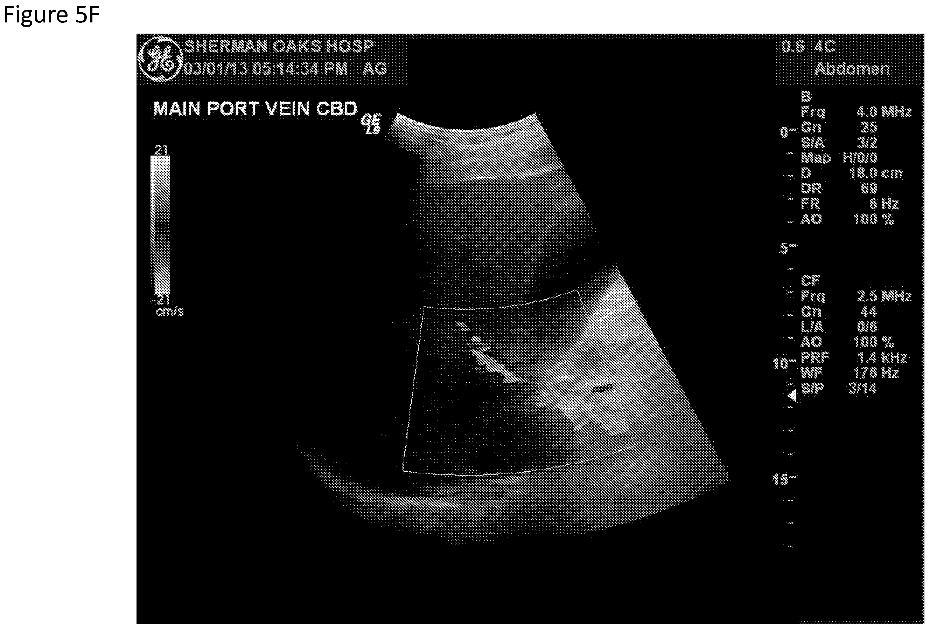

Figure 5A-5F depict, in accordance with an embodiment of the invention, ultrasound images of a gall bladder of a subject diagnosed with biliary colic, hypertension, and a significantly infected gall bladder. The subject received no treatment prior to the ultrasound.

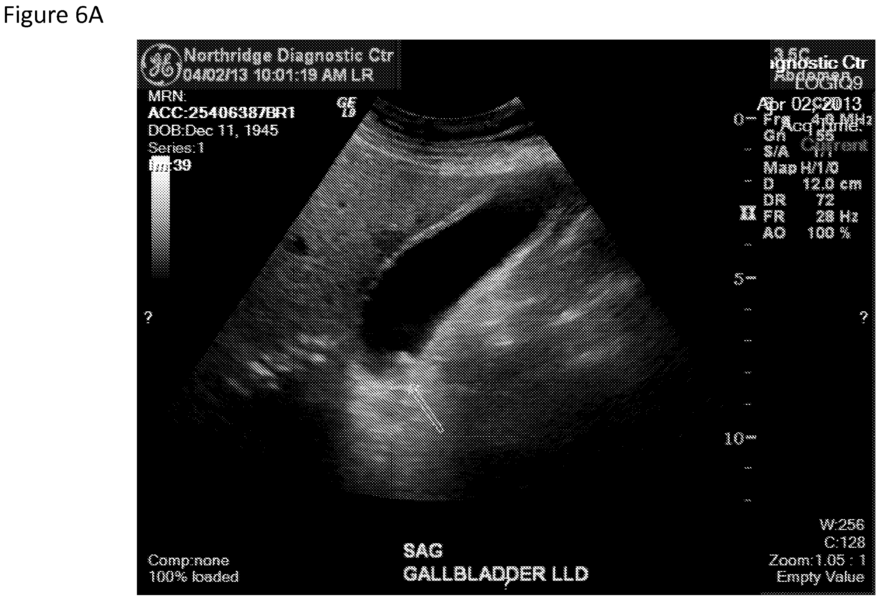

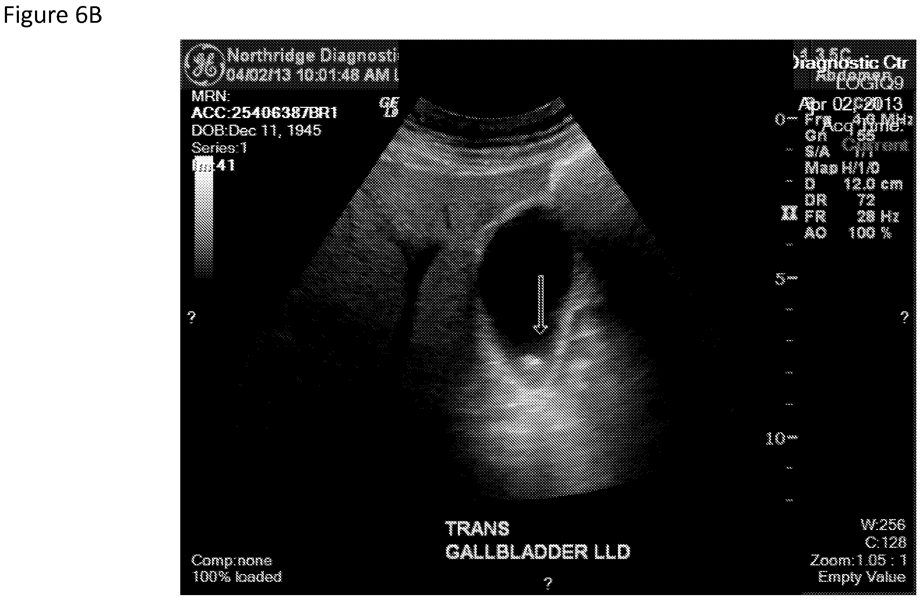

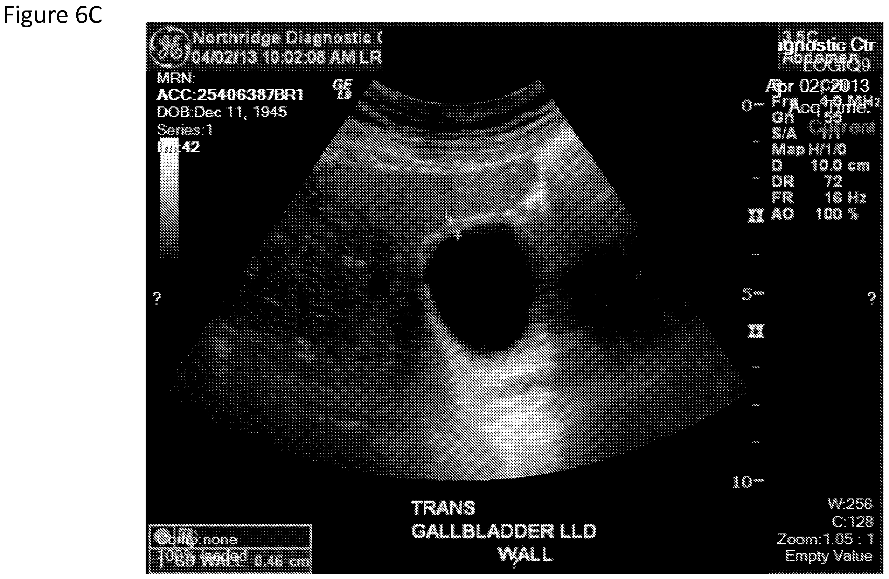

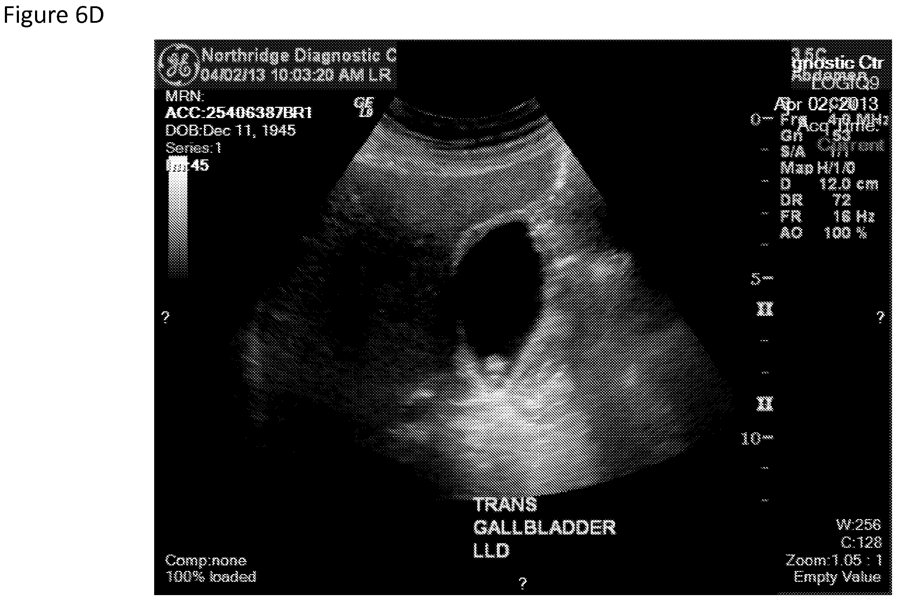

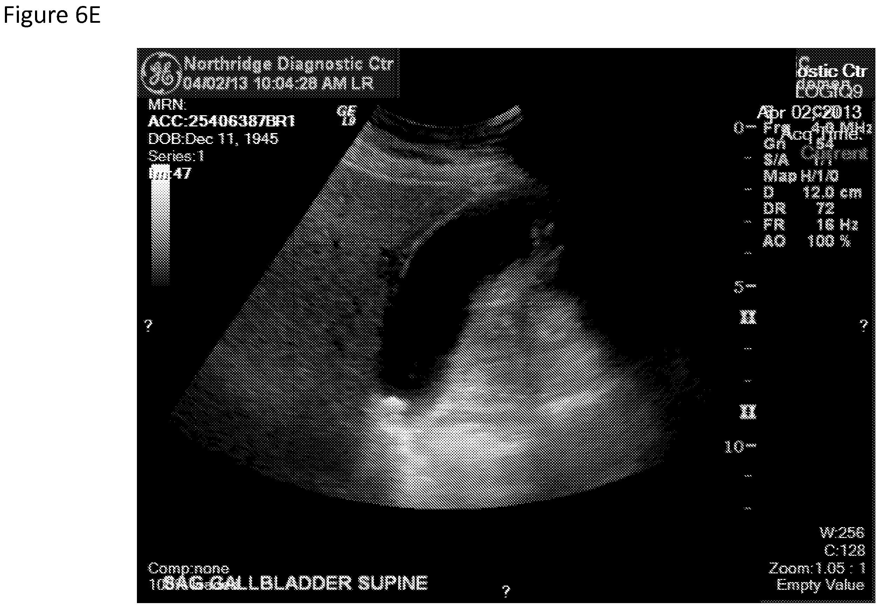

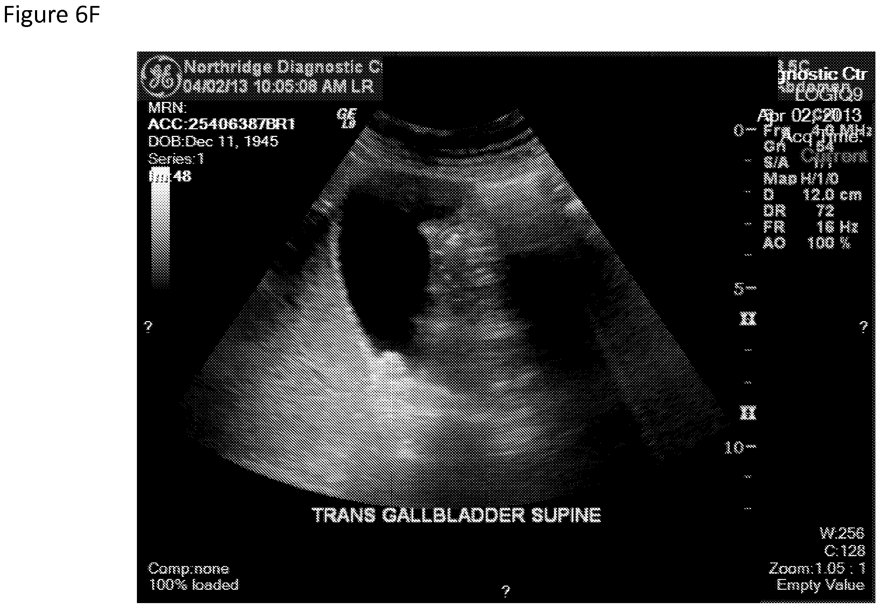

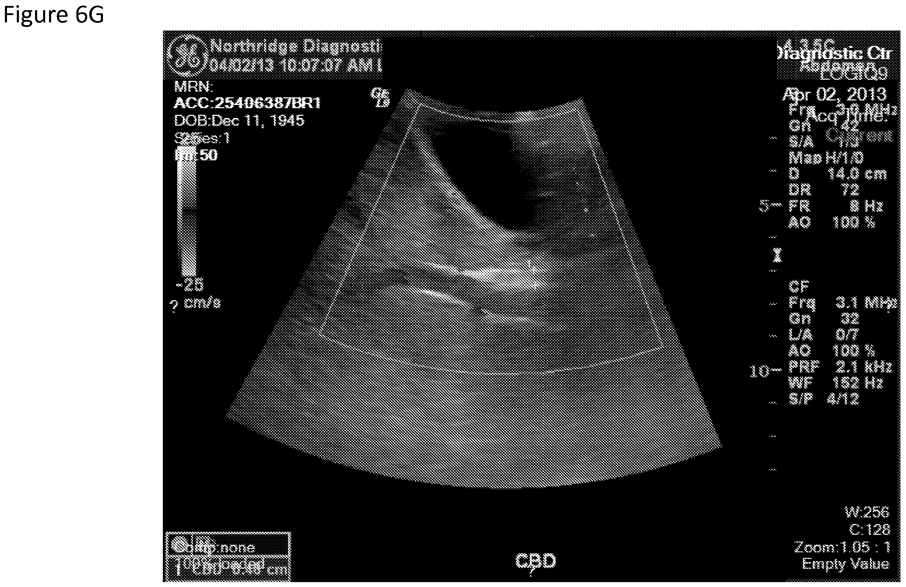

Figures 6A-6G depict, in accordance with an embodiment of the invention, ultrasound images of a gall bladder of the same subject shown in Figs. 5A-5F after the subject received a course of orally administered water containing water clusters.

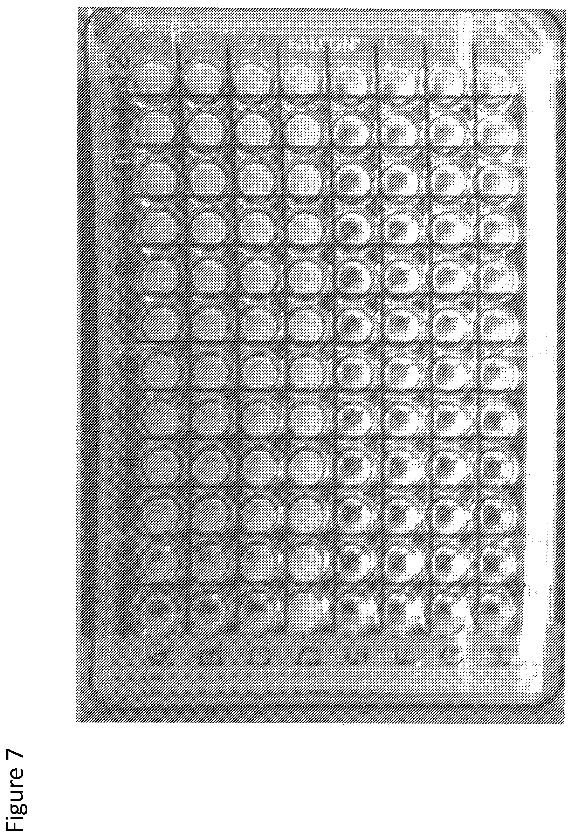

Figure 7 depicts, in accordance with an embodiment of the invention, *K. pneumoniae* treated against test samples 8x, 4x, & 1x (x = fold concentration increase of water clusters vs. standard formulation described herein). (Rows A-lx; B-4x; C-8x) and linezolid (Row D error should have been Ciprofloxacin control.) No efficacy was expected using a Gram positive antibiotic with a Gram negative pathogen. Worthy of note is that the water cluster containing formulation functions equally well with both Gram negative and Gram positive pathogens of which *K. pneumoniae* is a carbapenem resistant "superbug." MIC testing was performed according to the protocol described in the examples set forth herein, but with appropriate bacteria specific adjustments to the growth media.

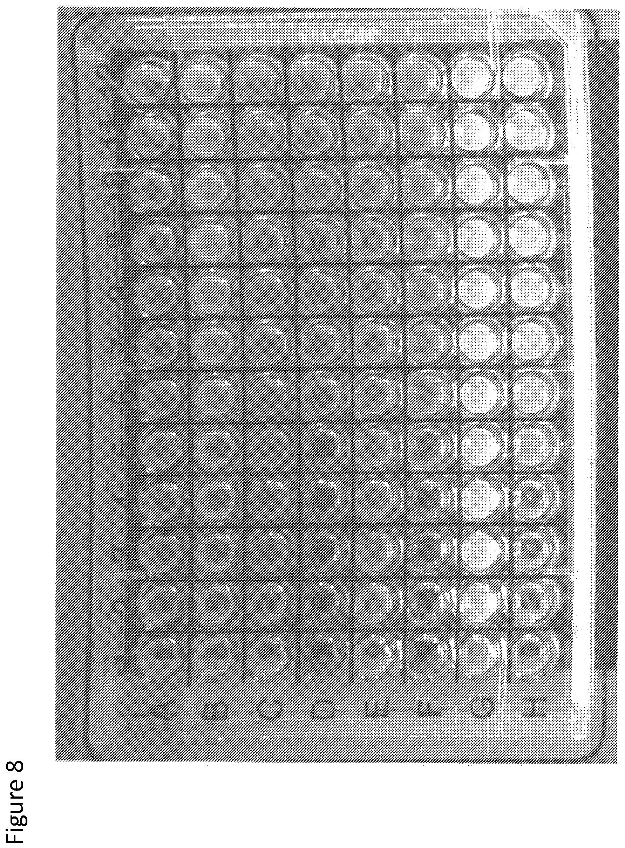

Figure 8 depicts, in accordance with an embodiment of the invention, *E.faecalis* treated against water cluster formulation (Row A-8x) and linezolid (Row B). *S. pyogenes* treated against water cluster formulation (Row C-8x) and linezolid (Row D). *S. sanguinis* treated against water cluster formulation (Row E-8x) and linezolid (Row F). *S. Aureus* VRSA treated against water cluster formulation (Row G-8x) and linezolid (Row H). In each case efficacy of the water cluster formulation is demonstrated by significant clearing. MIC testing was performed according to the protocols described in the examples set forth herein, with bacteria specific adjustments made to the growth media and antibiotics used.

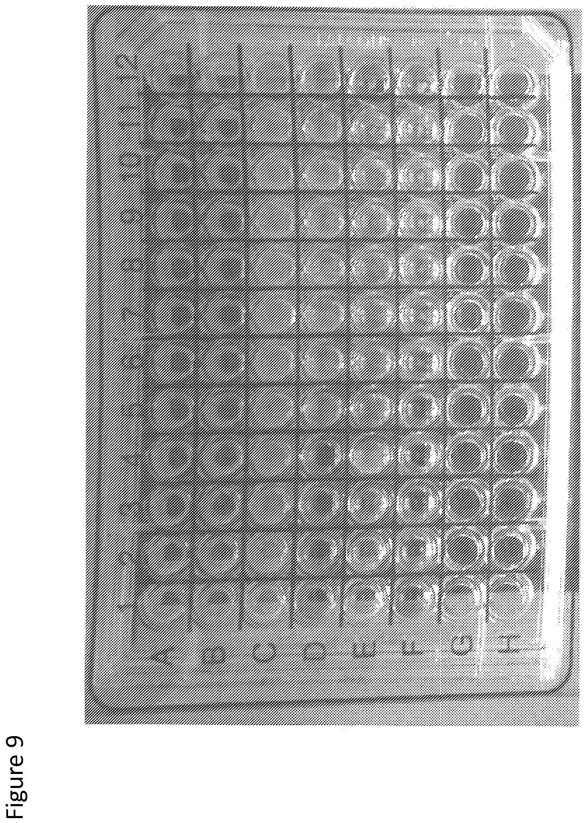

Figure 9 depicts, in accordance with an embodiment of the invention, *S. agalactiae* treated against water cluster composition (Row C-8x) and linezolid (Row D) and *S. mutans* treated against water cluster composition (Row E-8x) and linezolid (Row F). In each case efficacy of the water cluster formulation is demonstrated by significant clearing. MIC testing was performed according to the protocols described in the examples set forth herein, with bacteria specific adjustments made to the growth media and antibiotics used.

### DESCRIPTION OF THE INVENTION

Unless defined otherwise, technical and scientific terms used herein have the same meaning as commonly understood by one of ordinary skill in the art to which this invention belongs. Allen et al., Remington: The Science and Practice of Pharmacy 22nd ed., Pharmaceutical Press (September 15, 2012); Hornyak et al., Introduction to Nanoscience and Nanotechnology, CRC Press (2008); Singleton and Sainsbury, Dictionary of Microbiology and Molecular Biology 3rd ed., revised ed., J. Wiley & Sons (New York, NY 2006); Smith, March's Advanced Organic Chemistry Reactions, Mechanisms and Structure 7th ed., J. Wiley & Sons (New York, NY 2013); Singleton, Dictionary of DNA and Genome Technology 3rd ed., Wiley-Blackwell (November 28, 2012); and Green and Sambrook, Molecular Cloning: A Laboratory Manual 4th ed., Cold Spring Harbor Laboratory Press (Cold Spring Harbor, NY 2012), provide one skilled in the art with a general guide to many of the terms used in the present application. For references on pediatrics, see Schwartz et al., The 5-Minute Pediatric Consult 4th ed., Lippincott Williams & Wilkins, (June 16, 2005); Robertson et al., The Harriet Lane Handbook: A Manual for Pediatric House Officers 17th ed., Mosby (June 24, 2005); and Hay et al., Current Diagnosis and Treatment in Pediatrics (Current Pediatrics Diagnosis & Treatment) 18th ed., McGraw-Hill Medical (September 25, 2006).

One skilled in the art will recognize many methods and materials similar or equivalent to those described herein, which could be used in the practice of the present invention. Indeed, the present disclosure is in no way limited to the methods and materials described.

As used herein:
The acronym "IP" means intraperitoneal
The acronym "SC" means subcutaneous
The acronym "MSSA" means methicillin-sensitive *Staphylococcus aureus*
The acronym "MRSA" means methicillin-resistant *Staphylococcus aureus*
The acronym "SA" means *Staphylococcus aureus*

The term "prophylactic dose" means a dose that reduces the likelihood of acquiring an infection or developing a related condition, compared to the likelihood of acquiring an infection or developing a related condition if the prophylactic dose is not administered.

The term "water cluster," as used herein, refers to a solid state of a plurality of water molecules that are clustered together and stable at room temperature (whether suspended in H₂O solution or desiccated) and at normal atmospheric pressures. The water clusters described herein and non-limiting methods of making the same are described in S.Y. Lo et al., Evidence for the existence of stable-water-clusters at room temperature and normal pressure. Physics Letters A (2009), which is incorporated herein by reference in its entirety. Merely by way of non-limiting example, water clusters used in the inventive compositions and methods described herein can be formed by diluting NaCl into a concentration of 10⁻⁷ M in water with a particle count of 100 particles or less, no larger than 0.1 microns per mL. The water clusters described in the present application and utilized in conjunction with the inventive methods and kits described herein can exist and/or aggregate in a wide-range of sizes, from approximately 200 picometers to 7.5 nm. In some embodiments, the water clusters described in the present application and utilized in conjunction with the inventive methods and kits described herein can exist and/or aggregate in a size of up to several microns. Additionally, the water clusters can be present in relatively low to relatively high concentrations in the compositions described herein. Merely by way of example, when suspended in water, the water clusters may be present from approximately 1 million to over 1 billion water clusters per drop of water with an average water droplet size of 500 microns in diameter.

As used herein, a standard formulation of water clusters means water containing water clusters at a concentration of approximately 150 million water clusters per drop of water, with an average water droplet size of 500 microns in diameter. When describing concentrations, as used in the MIC testing set forth herein, the letter "x" means fold increase of water cluster concentration compared to a standard formulation of water clusters. Thus, for example, 8x means an 8 fold increase in water cluster concentration compared to a standard formulation.

"Animal" as used herein includes any known animal including but in no way limited to a mammal. "Mammal" as used herein refers to any member of the class *Mammalia,* including, without limitation, humans and nonhuman primates such as chimpanzees and other apes and monkey species; farm animals such as cattle, sheep, pigs, goats and horses; domesticated mammals, such as dogs and cats; laboratory animals including rodents such as mice, rats and guinea pigs, and the like. The term does not denote a particular age or sex. Thus, adult and newborn subjects, whether male or female, are intended to be included within the scope of this term. As indicated herein below, in some embodiments the compositions and methods described herein are intended for use on or with human subjects.

By way of additional background, the inventor determined that compositions that include water clusters (as depicted in Figs. 3 and 4) have antibacterial activity (as demonstrated in greater detail in the experiments set forth herein, and Figs. 1 and 2). The inventor also determined that a volume of water that includes water clusters can be evaporated at room temperature to leave water clusters behind (as demonstrated in the atomic force microscope images of Figs. 3 and 4, and described in the descriptions of those figures). Thus, substantially (or completely) anyhdrous water clusters can be re-suspended in a desired volume of water or other medium, in order to achieve a desired/effective water cluster concentration, as demonstrated in Fig. 1. In certain preferred compositions, the water in which the water clusters are suspended is ultrapure deionized water. In some embodiments, the water in which the water clusters are suspended also contains water clusters, and is prepared/purified according to the methodology set forth in S.Y. Lo et al., Evidence for the existence of stable-water-clusters at room temperature and normal pressure. Physics Letters A (2009)

Importantly, water clusters are known to form and exist in a range of sizes and shapes, depending upon the circumstances of their formation (e.g. the nature and quantity of dilute ions used) and the ambient environment. One of skill in the art would readily appreciate that for each of the embodiments described herein, water clusters formed from any type of very dilute ion in water could be used, and therefore the present invention is not limited to water clusters formed by diluting NaCl. Water clusters formed by significant dilution (as described above) of any known salt or ion described herein into any liquid substance described herein (including purified water, as described herein), or described in the references described or cited herein, may be used in conjunction with the inventive methods and kits.

The size of water clusters can be controlled by sonication, shaking, stirring vigorously, or otherwise agitating the liquid or semiliquid solution in which they are suspended. For certain applications, it may be advantageous to use compositions with smaller water clusters, while for other applications, larger water clusters may be more effective.

Based upon the experiments reported herein, the inventor believes the compositions described herein that include water clusters can be used for treating, preventing, promoting the prophylaxis of, and reducing the severity of bacterial infections. While certain specific applications of the compositions that include water clusters are provided herein below, the embodiments described are in no way intended to be limiting.

As described in greater detail in the examples provided herein below, certain tested inventive compositions that include water clusters demonstrate significant antibacterial activity against *S. mutans, S. Pneumonia,* and *S. Pyogenes.* Based upon these results, the inventor believes that compositions that include water clusters described herein are very likely effective as antibacterial agents against streptococcal pathogens and coccal pathogens in general. The inventor believes the various compositions described herein that include water clusters are likely also effective (when the appropriate concentration of water clusters are used) against a wide range of additional Gram-positive and Gram-negative bacteria, including but in no way limited to those bacteria described in Table 1.

The invention is described by reference to the claims.

**Table 1**

| **Bacteria** | **Genome** | **Gram** |
|---|---|---|
| Bacillus | B. anthracis | Gram-positive |
| Bacillus | B. cereus | Gram-positive |
| Bartonella | B. bacilliformis | Gram-negative |
| Bartonella | B. henselae | Gram-negative |
| Bartonella | B. quintana | Gram-negative |
| Bordetella | B. bronchisetica | Gram-negative |
| Bordetella | B. parapertussis | Gram-negative |
| Bordetella | B. pertussis | Gram-negative |
| Borrelia | B. burgdorferi sensu stricto | Gram-negative |
| Borrelia | B. garinii | Gram-negative |
| Brucella | B. melitensis | Gram-negative |
| Brucella | B. abortus | Gram-negative |
| Brucella | B. suis | Gram-negative |
| Brucella | B. canis | Gram-negative |
| Burkholderia | B. pseudomallei | Gram-negative |
| Burkholderia | B. mallei | Gram-negative |
| Campylobacter | C. jejuni | Gram-negative |
| Campylobacter | C. coli | Gram-negative |
| Capnocytophaga | C. canimorsus | Gram-negative |
| Chlamydia | C. trachomatis | Gram-negative |
| Chlamydia | C. pneumoniae | Gram-negative |
| Clostridium | C. botulinum | Gram-positive |
| Clostridium | C. difficile | Gram-positive |
| Clostridium | C. perfingens | Gram-positive |
| Clostridium | C. tetani | Gram-positive |
| Corynebacterium | C. diphtheria | Gram-positive |
| Corynebacterium | C. jeikeium | Gram-positive |
| Coxiella | C. burnetii | Gram-negative |
| Enterococcus | E. faecalis | Gram-positive |
| Enterococcus | E. faecium | Gram-positive |
| Escherichia | E. coli | Gram-negative |
| Fusobacterium | F. novum | Gram-neeative |
| Haemophilus | H. influenzae | Gram-negative |
| Helicobacter | H. pylori | Gram-negative |
| Klebsiella | K. pneumoniae | Gram-neeative |
| Legionella | L. pneumophila | Gram-negative |
| Listeria | L. monocytogenes | Gram-positive |
| Mycobacterium | M. tuberculosis | Gram-positive |
| Mycobacterium | M. leprae | Gram-positive |
| Mycobacterium | M. ulcerans | Gram-positive |
| Mycoplasma | M. pneumoniae | Gram-positive |
| Neisseria | N. gonorrhoeae | Gram-negative |
| Neisseria | N. meningitidis: | Gram-negative |
| Pasteurella | P. multocida | Gram-negative |
| Pseudomonas | P. aeruginosa | Gram-negative |
| Salmonella | S. typhimurium | Gram-negative |
| Salmonella | S. typhi | Gram-negative |
| Shiigella | S. dysenteriae | Gram-negative |
| Shiigella | S. flexneri | Gram-negative |
| Shiigella | S. boydii | Gram-negative |
| Shiigella | S. sonnei | Gram-negative |
| Staphylococcus | S. aureus | Gram-positive |
| Streptococcus | S. pyogenes | Gram-positive |
| Streptococcus | S.agalactiae | Gram-positive |
| Streptococcus | S. pneumoniae | Gram-positive |
| Vibrio | V. cholerae | Gram-negative |
| Yersinia | Y. pestis | Gram-negative |
| Yersinia | Y. pseudotuberculosis | Gram-negative |
| Yersinia | Y. enterocolitica | Gram-negative |

### Antibacterial applications in general

As demonstrated in the experiments described in the examples set forth herein, water clusters have antibacterial properties, which, while not wishing to be bound by any one particular theory, likely relate to interfering with the integrity of the bacterial cell wall, especially during bacterial reproduction. Therefore the inventor believes that the various compositions described herein that include water clusters can be used as an antibacterial substance to treat or prevent Gram-negative and Gram-positive bacterial infections, by directly applying a composition including water clusters on a subject (as described in greater detail herein below), and/or by applying a composition including water clusters to any environment or object with which a subject may interact. In certain embodiments, the composition that includes water clusters is liquid water. In certain embodiments, the composition that includes water clusters is purified water with a particle count (particles ranging from 0.1 microns - 0.5 microns as determined by a laser particle counter) of less than 10,000 counts per 1mL. In some embodiments, the composition that includes water clusters is purified water with a particle count of less than 9,000 counts per 1mL, or less than 8,000 counts per 1 mL, or less than 7000 counts per 1mL, or less than 6000 counts per 1mL, or less than 5,000 counts per 1mL, or less than 4,000 counts per 1mL, or less than 3,000 counts per 1mL, or less than 2000 counts per 1 mL, or less than 1000 counts per 1mL, or less than 500 counts per 1mL, or less than 100 counts per 1 mL, or less than 10 counts per 1 mL. In some embodiments the composition that includes the water clusters is water characterized by 18.2MΩcm resistivity. In some embodiments, the composition that includes the water clusters is water characterized by 16-20MΩcm resistivity. In some embodiments, the composition that includes the water clusters is deionized water. In some embodiments, the composition that includes the water clusters is tap water. In some embodiments, the composition that includes the water clusters is water that has been treated by the process of reverse osmosis. The particle counts described above refer to those of the liquid water in which the water clusters are formed or added, and the particle count doesn't include the water clusters themselves.

It has been demonstrated that water clusters effectively treat infections on the skin of a human when applied topically using a water-based composition. Based on human testing, water clusters (provided in various formulations described herein) also effectively treat a bacterial infection residing inside of the body, on an exterior surface of the body, and in mucous membranes, when administered orally. Greater detail regarding routes of administration, specific compositions, and diseases that are likely to be treatable using compositions that include water clusters are provided herein below.

Because it has been determined compositions that include water clusters are effective at killing and reducing the growth rate of bacteria, it is believed that an antibacterial effect will be achieved if they are placed in contact with bacteria residing (transiently or more permanently) on any surface. In other words, it is believed that water clusters may exert an antibacterial effect when applied to any non-living material in which bacterial growth or occupancy is not desired, either by reducing an existing bacterial population (in a biofilm or otherwise), or by preventing or slowing the growth of bacteria. The inventor believes that the water clusters are especially effective when hydrated (e.g. in a liquid, gel, foam, or other compound described herein).

### Controlling bacterial colonization of an environment associated with food and non-edible plants

In addition to the embodiments described above, also provided are methods for using compositions suitable for controlling (reducing or eliminating) bacterial colonization of an environment capable of sustaining or supporting microbial growth. As indicated above, the term "colonization" as used herein, includes colonization of every form, including colonization typically associated with a biofilm.

Accordingly, the disclosure teaches a method for utilizing a water cluster containing composition for reducing the overall bacterial count or population in an environment by applying to the environment an effective amount of the foregoing composition. One non-limiting example of an environment includes food products. The term "food" or "food product" encompasses all edible nutritive substances and compositions, including those intended for human consumption as well as pet or other animal consumption, such as pet food animal feed, etc. "Pet food" means a composition that is intended for ingestion by the pet. Pet food compositions may include, without limitation, nutritionally balanced compositions suitable for daily feed, as well as supplements (e.g., treats and edible films) which may or may not be nutritionally balanced.

In practice, the compositions can be applied to a food in combination with one or more sublethal processing treatments such as sublethal heat treatment. In preferred aspects, relatively low heat can be used due to the efficacy of the compositions that include water clusters. In other aspects, the water cluster containing compositions can be used as food additives to combat foodborne pathogens. In some aspects, the compositions can be used as feed additives, to be used to improve the health of farm animals and/or for the prevention or treatment of diseases caused by microbes (bacteria as a source of a primary, secondary, or higher order infection, and other pathogens, by way of supporting the animal's immune system, directly or indirectly, as described herein).

Merely by way of example, the compositions can be encapsulated using a carrier or excipient as described herein below and then added to a feed by any known means of applying it to feed such as for example, by mechanical mixing, spraying, etc.

In some examples, the composition can be mixed with any granular component that is digestable, such as for example, milled maize grain; ground grains such as for example oats, wheat, buckwheat; ground fruits such as for example, pears, etc. The composition is then added to any type of animal feed in amounts effective to either kill, or inhibit the growth or activity of susceptible bacteria. Examples of animal feed include, but are not limited to, green foder, silages, dried green fodder, roots, tubers, fleshy fruits, grains, seeds, brewer's grains, pomace, brewer's yeast, distillation residues, milling byproducts, byproducts of the production of sugar, starch or oil production, and various food wastes. The product can be added to the animal feedstuffs for cattle, poultry, rabbit, pig, or sheep rearing, etc. It can be used mixed with other feed additives for these stock.

In yet other aspects, the compositions that include water clusters can be used as agricultural control agents to control plant and soil pathogens by applying an effective amount of a composition including water clusters to a plant and/or the soil in which it resides. Formulations comprising water clusters can be applied by crop
dusting or by using any other traditional means of applying a pesticide to a crop. If dried or semi-dried formulations are used, they will be re-hydrated when the crops are watered or when it rains, and therefore a certain percentage of the composition (and therefore water clusters) will be introduced into the soil. Alternatively, any version of the compositions described herein (dried, semi-dried, gas, liquid, and combinations thereof) can be introduced directly into the soil.

In an additional aspects, the present disclosure provides an agricultural crop or other plant having a reduced live bacterial population as a result of having applied thereon an effective amount of the foregoing antibacterial composition.

In addition to the application on crops, as described above, the present disclosure also teaches application of the compositions on non-edible plants, or plants only edible to nonhuman animals that are traditionally not regarded as crops.

An "effective amount" as used in this context of treating plants means an amount effective for inhibiting plant pathogen development, or for eliminating any existing plant pathogen already found on or in the plant.

### Treatment of non-biological or inert surfaces or substrates

In some aspects, the disclosure teaches methods of applying antibacterial water cluster compositions described herein to non-biological or inert surfaces or substrates. In some aspects, the compositions described herein can be applied to said substrates in liquid, solid, atomized or nebulized form. Any surface upon which bacteria are known to reside, even temporarily, is contemplated as a surface that can be treated according to this aspect of the disclosure.

Merely by way of non-limiting examples, the compositions can be applied or impregnated on any device, instrument, dressing (or other fabric) used and/or administered and/or prescribed in a medical or non-medical setting for use on or in any animal, including humans, by any method known in the art.

Non-limiting examples of wound care devices on or in which the compositions could be applied or impregnated include a non-resorbable gauze/sponge
dressing, a hydrophilic wound dressing, an occlusive wound dressing, a hydrogel wound dressing, a foam dressing, a burn dressing, and the like.

Non-limiting examples of instruments and devices upon which the water cluster containing compositions can be included are disposable or permanent or indwelling catheters, long term urinary devices, tissue bonding urinary devices, wound drain tubes, ventricular catheters, endotracheal tubes, breathing tubes, feeding tubes, dairy lines, drinking water lines and the like.

In some aspects, a substantially powdered, liquid, atomized, or nebulized formulation that includes water clusters can be applied to or impregnated on one or more coponents of a heating, ventilation, and air conditioning (HVAC) system. In some aspects, the compositions can be applied to one or more filter of an HVAC system. In some aspects, the compositions can be applied to one or more intake components of an HVAC system. In some aspects, the compositions can be applied to one or more components for delivering air cleaned by the HVAC system. One of skill in the art would readily appreciate that the water clusters could be widely disseminated into a desired environment using an HVAC system that includes the water clusters described above.

In some aspects, the compositions can be used (in liquid or solid form) as a disinfectant that can be applied to a surface in any manner known, including but in no way limited to spraying, pouring, powdering, fumigating, and the like. Merely by way of non-limiting examples, the compositions can be applied to surfaces such as tabletops, countertop, bathtub, tile, and the like. Additional non-limiting examples of surfaces upon which the compositions may be applied include pipelines in industries such as food and beverage industries, paper mills, cooling towers, and gas and oil industries. Compositions can be advantageously combined with common household disinfectants, or any other disinfectant typically used in the aforementioned settings.

For the purposes of impregnating any media described herein, the water cluster containing compositions can be incorporated into a substance that is chemically bonded to said media, or that is temporarily affixed to said media (i.e. stuck to the medium using a sticky substance that acts as an adhesive on at least one side).

### Treatment of animal subjects

In some embodiments, a composition including the water clusters described herein can be administered by any route of administration described herein for the purpose of reducing bacterial burden, or the likelihood of bacterial infection, in an individual who has another type of infection. In these instances, the compositions including the water clusters described herein are essentially used to treat or prevent a secondary (or higher order) bacterial infection in an individual already infected with another organism. In some embodiments, the other organism is any known pathogen, whether that pathogen is directly treatable by any composition and/or route of administration described herein or not.

In some embodiments of the invention, the compositions that include water clusters are combined with other antimicrobial substances (e.g. antibiotic, anti-biofilm, antiviral, antifungal, antiparasitic, etc.) in a single formulation. In some embodiments, rather than combining water clusters with any of the aforementioned antimicrobial substances into a single formulation, a composition in which water clusters are suspended (such as any composition described herein) is administered separately from any of the aforementioned antimicrobials, either contemporaneously, or at a different time, according to a predetermined treatment regimen. In certain embodiments, oral formulations that include water clusters are administered to a subject along with a traditionally recommended dose of any of the aforementioned antimicrobials, as part of a treatment regimen. In other embodiments, lower doses of antimicrobials are administered to a subject (compared to usual dosages) when administered in conjunction with the inventive compositions that include water clusters.

Compositions that include water clusters can be administered anywhere on a subject's skin or mucous membranes, ingested orally, inhaled by using a nebulizer, atomizer or like device, and administered into any orifice of a subject's body. Likewise, a composition comprising water clusters can be administered as a rinse in any orifice or lumen in a subject's body, including, but in no way limited to an orifice or lumen included in or associated with a subject's nose, mouth, vagina, penis, ear, and anus. A composition comprising water clusters can also be used to clean an area surrounding a point of entry for a surgical instrument, thereby reducing the number of viable bacteria introduced into a patient by the instrument. A composition comprising water clusters can also be administered into any other non-naturally occurring orifice in a subject's body. Merely by way of example, an orifice used for introducing one or more fluid or gas into or removing one or more fluid or gas from a subject's body. Merely by way of example, an opening surgically introduced into a subject's body during a tracheostomy, or for the purposes of drainage (i.e. an opening suitable for a shunt or catheter), could be used as an entry point for administering any composition described herein.

In certain embodiments, the disclosure includes a method for treating wounds by administering a therapeutically effective amount of a composition of the invention that includes water clusters. In some embodiments, the wounds can include, but are in no way limited to, cutaneous abscess, surgical wounds, sutured lacerations, contaminated lacerations, burn wounds such as partial and full thickness burns, decubitus ulcers, stasis ulcers, leg ulcers, foot ulcers, venous ulcers, diabetic ulcers, ischemic ulcers, and pressure ulcers.

Importantly, any method of providing the compositions and/or administering them to a subject by any route described herein is within the scope of the disclosure.

In various embodiments, the water cluster containing compositions of the present disclosure can be provided as pharmaceutical compositions including a pharmaceutically acceptable excipient. "Pharmaceutically acceptable excipient" means an excipient that is useful in preparing a pharmaceutical composition that is generally safe, non-toxic, and desirable, and includes excipients that are acceptable for veterinary use as well as for human pharmaceutical use. Such excipients may be solid, liquid, semisolid, or, in the case of an aerosol composition, gaseous.

In various embodiments, the pharmaceutical compositions used according to the invention may be formulated for delivery via any route of administration. In some embodiments, the present invention includes providing and/or administering the water cluster containing compositions described herein to a subject by any route of administration. "Route of administration" may refer to any administration pathway known in the art, including but not limited to aerosol, nasal, oral, transmucosal, transdermal or parenteral. "Transdermal" administration can be accomplished using a water-based formulation, or a topical cream or ointment or by means of a transdermal patch. "Parenteral" refers to a route of administration that is generally associated with injection, including but in no way limited to intraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal. Via the parenteral route, the compositions may be in the form of (but is in no way limited to) solutions or suspensions for infusion or for injection, or as lyophilized powders. Via the enteral route, the pharmaceutical compositions may be in the form of tablets, gel capsules, sugar-coated tablets, syrups, suspensions, solutions, powders, granules, emulsions, microspheres or nanospheres or lipid vesicles or polymer vesicles allowing controlled release. Via the parenteral route, the compositions may be in the form of solutions or suspensions for infusion or for injection. Via the topical route, the pharmaceutical compositions containing water clusters used according to the invention can be formulated for treating the skin and mucous membranes and are in the form of ointments, creams, milks, salves, powders, impregnated pads, solutions, gels, sprays, lotions or suspensions, and the like. They can also be in the form of microspheres or nanospheres or lipid vesicles or polymer vesicles or polymer patches and hydrogels allowing controlled release. These topical-route compositions can be either in anhydrous form or in aqueous form depending on the clinical indication.

The pharmaceutical compositions used according to the invention can also contain any pharmaceutically acceptable carrier. "Pharmaceutically acceptable carrier" as used herein refers to a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or a combination thereof. Each component of the carrier must be "pharmaceutically acceptable" in that it must be compatible with the other ingredients of the formulation. It must also be suitable for use in contact with any tissues or organs with which it may come in contact, meaning that it must not carry a risk of toxicity, irritation, allergic response, immunogenicity, or any other complication that excessively outweighs its therapeutic benefits.

The pharmaceutical compositions that include clustered water used according to the invention can also be encapsulated, tableted or prepared in an emulsion or syrup for oral administration. Pharmaceutically acceptable solid or liquid carriers may be added to enhance or stabilize the composition, or to facilitate preparation of the composition, or to enhance a biofoam composition performance (such as enzymes etc.). Liquid carriers include, but are in no way limited to, syrup, peanut oil, olive oil, glycerin, saline, alcohols and water. Solid carriers include starch, lactose, calcium sulfate, dihydrate, terra alba, magnesium stearate or stearic acid, talc, pectin, acacia, agar or gelatin. The carrier may also include a sustained release material such as glyceryl monostearate or glyceryl distearate, alone or with a wax.

The pharmaceutical preparations are made following the conventional techniques of pharmacy involving milling, mixing, granulation, and compressing, when necessary, for tablet forms; or milling, mixing and filling for hard gelatin capsule forms. When a liquid carrier is used, the preparation may be in the form of a syrup, elixir, emulsion or an aqueous or non-aqueous suspension. Such a liquid formulation may be administered directly p.o., filled into a soft gelatin capsule, or via any other means known in the art.

The pharmaceutical compositions used according to the invention may be delivered in a therapeutically effective amount. The precise therapeutically effective amount is that amount of the composition that will yield the most effective results in terms of efficacy of treatment in a given subject. This amount will vary depending upon a variety of factors, including but not limited to the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. One skilled in the clinical and pharmacological arts will be able to determine a therapeutically effective amount through routine experimentation, for instance, by monitoring a subject's response to administration of a compound and adjusting the dosage accordingly. For additional guidance, see Remington: The Science and Practice of Pharmacy (Gennaro ed. 20th edition, Williams & Wilkins PA, USA) (2000).

Typical dosages of an effective amount of the water cluster containing composition can be as indicated to the skilled artisan by the *in vitro* responses or responses in animal models. Such dosages typically can be reduced by up to about one order of magnitude in concentration or amount without losing the relevant biological activity. Thus, the actual dosage will depend upon the judgment of the physician or other expert, the condition of the subject, and the effectiveness of the therapeutic method.

When applied topically to treat a bacterial infection, a therapeutic dosage may be .0001 - 10mL of water (or more) per infected square millimeter of tissue (at approximately 1 million - 1 billion water clusters per drop of water with an average water droplet size of 500 microns in diameter), applied 1 - 100 times per day (or continuously, by constantly re-applying a wet dressing or otherwise) for from 1 - 100 days, or until the bacterial infection resolves.

When applied topically to prevent or promote the prophylaxis of a bacterial infection, a prophylactic dosage may be .0001 -10 mL of water per square millimeter of treatable tissue (at approximately 1 million - 1 billion water clusters/drop of water with an average water droplet size of 500 microns in diameter), applied 1 - 100 times per day (or continuously, by constantly re-applying a wet dressing or otherwise) from 1 - 100 days, or until the perceived risk of bacterial infection is reduced significantly or eliminated.

When administered orally to treat a bacterial infection, the therapeutic dosage may be .001 - 50 mL of water per kg of body weight per day (at approximately 1 million - 1 billion water clusters per drop of water with an average water droplet size of 500 microns in diameter), ingested at from 1 - 50 times per day (to achieve a desired dosage in the above range) for from 1 - 100 days, or until the bacterial infection resolves.

When administered orally to prevent or promote the prophylaxis of a bacterial infection, the therapeutic dosage may be .001 - 50 mL of water per kg of body weight per day (at approximately 1 million - about 1 billion water clusters per drop of water with an average water droplet size of 500 microns in diameter), ingested 1 - 50 times per day (to achieve a desired dosage in the above range) for from 1 - 100 days, or until the perceived risk of a bacterial infection is reduced or significantly eliminated.

The present disclosure is also directed to a kit to treat and/or prevent a pathogenic infection in an animal (including mammal - e.g. human) in need thereof. The kit is useful for practicing the method of treating and/or preventing a pathogenic infection, in particular an infection caused by a bacterial pathogen. The infection may be caused by any organism described herein, and includes organisms responsible for infections that may be secondary or primary to a bacterial infection treated by the compositions and methods. The kit is an assemblage of materials or components, including at least one of the compositions. Thus, the kit contains a composition that includes water clusters.

In some aspects, a kit includes a quantity of an composition described herein that includes water clusters, any suitable applicator device or material for applying said composition, and optionally instructions for the use thereof.

In some aspects, a kit includes a quantity of any material described herein upon or in which an composition comprising water clusters has been impregnated or applied by any means.

The exact nature of the components configured in the kit depends on its intended purpose. For example, some aspects are configured for the purpose of treating a bacterial infection in a living organism (plant or animal). Other aspects are configured for prophylaxis. In one aspect, the kit is configured particularly for the purpose of treating mammalian subjects. In another aspect, the kit is configured particularly for the purpose of treating human subjects. In another aspect, the kit is configured for treating adolescent, child, or infant human subjects. In further aspects the kit is configured for veterinary applications, treating subjects such as, but not limited to, farm animals, domestic animals, and laboratory animals. In yet other aspect, the kit is configured for applying the compositions to crops, other foodstuffs, or other plants.

Instructions for use may be included in the kit. "Instructions for use" typically include a tangible expression describing the technique to be employed in using the components of the kit to effect a desired outcome, such as to treat and/or prevent an infection caused by a bacterial pathogen. Optionally, the kit also contains other useful components, such as, diluents, buffers, pharmaceutically acceptable carriers, syringes, catheters, applicators, pipetting or measuring tools, bandaging materials, spraying devices or other useful paraphernalia, depending upon the intended application, as will be readily recognized by those of skill in the art.

The materials or components assembled in the kit can be provided to the practitioner stored in any convenient and suitable ways that preserve their operability and utility. For example the components can be in suspended, dissolved, dehydrated, or lyophilized form; they can be provided at room, refrigerated or frozen temperatures. The components are typically contained in suitable packaging material(s). As employed herein, the phrase "packaging material" refers to one or more physical structures used to house the contents of the kit, such as inventive compositions comprising water clusters and the like. The packaging material is constructed by well-known methods, preferably to provide a sterile, contaminant-free environment. As used herein, the term "package" refers to a suitable solid matrix or material such as glass, plastic, paper, foil, and the like, capable of holding the individual kit components. Thus, for example, a package can be one or more glass vials or plastic containers used to contain suitable quantities of an composition that includes water clusters. The packaging material generally has an external label which indicates the contents and/or purpose of the kit and/or its components.

### EXAMPLES

### Experiments I

### Example 1

### Materials and Methods

Minimum inhibitory concentration assay (MIC) of ultrapure water that includes water clusters (hereafter "water cluster composition") was tested on *Streptococcus pneumonia* using the methodology from Clinical and Laboratory Standards Institute's approved standard, M7-A7.

### Formulation

A test sample of water containing water clusters at a concentration of approximately 150 million water clusters per drop of water, with an average water droplet size of 500 microns in diameter, (prepared according to the methods described in S.Y. Lo et al., Evidence for the existence of stable-water-clusters at room temperature and normal pressure. Physics Letters A (2009)) was stored at room temperature until required. Linezolid was diluted in dimethyl sulfoxide to 10 mg/mL and was used as a control for MICs.

### MIC drug master plate

The test sample of water containing water clusters (as described above) was added to the first well of a 96- well plate. 2-fold dilutions were performed into all the wells of that row containing the appropriate broth for *Streptococcus pneumoniae* (*S. pneumonia*) ATCC 49619 except for the last well, which was used as a control without any testing agent. Cation-adjusted Mueller-Hinton broth (CA-MHB) with 3% lysed horse blood was used. Dilutions of the Linezolid control were prepared similarly, except they were diluted to 8 µg/mL in the first well, followed by 2-fold serial dilutions into the broth for *S. pneumonia.*

### Bacterial strains

*S. pneumoniae* ATCC 49619 was obtained from the American Type Culture Collection (Manassas, VA). This strain was maintained as a frozen glycerol stock at -80°C. A working stock was prepared by streaking *S. pneumoniae* onto an appropriate agar plate, incubating it at 35°C for 24 hours and suspending isolated colonies in 3mL of 0.9% sterile saline solution to approximately 1 x 10⁸ CFU/mL. *S. pneumoniae* was grown in the presence of 5% CO₂.

### MIC assay

A bacterial working stock was diluted into appropriate liquid media for a final inocula of about 5 x 10⁶ CFU/mL. 10 µL of stock suspension was added to each well containing the serial dilutions described above. The test articles from the drug master plate were added to the rows of each MIC plate, rows A-C and E-G testing against the water cluster composition, while row H tested against Linezolid. All wells were inoculated within 15 minutes of turbidity adjustment of the stock suspension. The plates were incubated at 35°C for 20-24 hours prior to reading the results.

The water cluster composition was also dehydrated in both glass and plastic. They were then rehydrated in the same volume of sterile saline and mixed well using a vortex and orbital shaker. The MIC was performed against *S. pneumoniae* ATCC 49619. Additionally, 2 mL of water cluster composition was dehydrated in both glass and plastic and rehydrated in 200 µL of sterile water. This was also tested using the MIC assay.

### Example 2

### Results

### Discussion and conclusion

The water cluster composition was tested against *S. pneumoniae* ATCC 49619 using an MIC assay. *S. pneumoniae* ATCC 49619 was tested 3 times using water cluster composition before dehydration, after dehydration in glass and after dehydration in plastic, both rehydrated in the same volume of sterile water. MIC of the water cluster composition tested against *S. pneumoniae* was 90% (10 µL of stock solution added to 90 µL of water cluster composition).

### Reference cited in testing report

Clinical and Laboratory Standards Institute. Performance Standards for Antimicrobial Susceptibility Testing: Twenty-first Informational Supplement M100-S21. Wayne, PA, USA: CLSI; 2011.

### Experiments II

### Example 3

### Materials and Methods

### Formulation

A test sample of water containing water clusters at a concentration of approximately 150 million water clusters per drop of water, with an average water droplet size of 500 microns in diameter, was stored at room temperature until required. The sample of water was prepared according to the methods described in S.Y. Lo et al., Evidence for the existence of stable-water-clusters at room temperature and normal pressure. Physics Letters A (2009)). Linezolid diluted in dimethyl sulfoxide was used as a control.

### Drug master plate

The test sample of water containing water clusters was added to the first well of a 96-well plate. 2-fold dilutions were performed into all of the wells of that row containing cation-adjusted Mueller-Hinton Broth (CA-MHB) with 5% lysed horse blood except for the last well, which was used as a control without any testing agent. This was repeated in Brain Heart Infusion (BHI) broth. The Linezolid control followed a similar protocol but was diluted to 8µg/mL in the first well, followed by 2-fold serial dilutions into both CA-MHB with 5% lysed horse blood and BHI broth.

### Bacterial strains

*Streptococcus mutans* ATCC 31341, *Streptococcus pneumonia* ATCC 49619, and *Streptococcus pyogenes* ATCC 51339 were obtained from the American Type Culture Collection (Manassas, VA). All strains were maintained as frozen glycerol stocks at -80°C. Working stocks were prepared by streaking bacteria onto Sheep Blood Agar plates, growing at 37°C with 5% CO₂ for 24 hours and suspending isolated colonies in 3mL of 0.9% sterile saline to approximately 1 x 10⁸ CFU/mL.

### MIC assay

Bacterial working stocks were diluted into appropriate liquid media for a final inocula of about 5 x 10⁶ CFU/mL. CA-MHB with 5% lysed horse blood was used for all the *Streptococcus spp.* While BHI broth was also used for *S. mutans.* 10µL of stock suspension was added to each well of a 96-well MIC plate treated with the test sample of water containing water clusters. The test articles from the drug master plate were added to the rows of each MIC plate, the first four rows testing against the water cluster composition, while the last four rows were tested against Linezolid. All wells were inoculated within 15 minutes of turbidity adjustment of the stock suspension. This was performed in quadruplicate. The plates were incubated at 35°C with 5% CO₂ for a full 24 hours before reading.

### Results

The MIC data can be found in Table 2 and supported by Figure 2. For the *Streptococcus spp.,* there was visually no turbidity in the first well within the first 24 hours. The Linezolid control was within the published range for the tested strains.

**Table 2. Minimal inhibitory concentrations of test formulation and control antibiotic**

| **Organism** | **Water cluster composition** | **Linezolid** |
|---|---|---|
| *S. mutans* ATCC 31341 | 90% | 1µg/mL |
| *S. pneumonia* ATCC 49619 | 90% | 1µg/mL |
| *S. pyogenes* ATCC 51339 | 90% | 1µg/mL |

### Discussion

The water cluster composition was tested against 3 bacterial strains: *Streptococcus mutans* ATCC 31341, *Streptococcus pneumoniae* ATCC 49619, and *Streptococcus pyogenes* ATCC 51339 using an MIC assay. Serial dilutions were used. Visually, the first well with 90% water cluster composition was clear after 24 hours of growth in all plates, as exemplified by the plate for *S. pneumonia* shown in Figure 2.

### Experiments III

### Example 4

### Materials and Methods

A test sample of water containing water clusters at a concentration of approximately 150 million water clusters per drop of water, with an average water droplet size of 500 microns in diameter, was stored at room temperature until required. The sample of water was prepared according to the methods described in S.Y. Lo et al., Evidence for the existence of stable-water-clusters at room temperature and normal pressure. Physics Letters A (2009)). An aliquot of the test sample of water was placed onto a glass slide cleaned with ultra-pure water and allowed to evaporate, leaving a residue behind for analysis. A sample of the residue was imaged with Atomic Force Microscopy (AFM) in the tapping mode (Figs. 3 and 4). AFM images provide a view of the texture of the sample surface. AFM images are presented with a Z-scale which is greatly exaggerated with respect to the X and Y (plane of the sample) scales. The AFM scan was performed in a 20 µm x 20 µm and a 5 µm x 5 µm area. The analysis areas were selected optically using low magnification. The images presented in Figs. 3 and 4 were processed with a 1st order flatten routine to remove sample tilt.

### Experiments IV

### Example 5

### Treatment of MRSA Infection

A 63 year old otherwise healthy male subject presented with an unsealed highly inflamed hole with a depth of several millimeters in his arm. The hole was approximately the width of a standard pencil, and the subject was diagnosed as having MRSA. The infection had not been successfully treated by standard means. The infection was diagnosed as likely resulting from a patch of cellulitis on the subject's forearm. The subject's wound was treated by using a saturated cotton ball to apply water containing water clusters at a concentration of approximately 150 million water clusters per drop of water, with an average water droplet size of 500 microns in diameter. After day 1 of treatment, redness was reduced and weeping of the wound stopped. After day 2 of treatment, the hole in the subject's arm began to fill with a white substance. After day 3 of treatment, the hole was almost completely filled in, and inflammation was significantly reduced. After day 4 of treatment, there was no visible inflammation and the hole was sealed completely with new skin. After day 5 of treatment the tissue had returned to a normal shape and skin texture was almost normal. Treatment was stopped at day 6 with no recurrence of infection.

### Experiments V

### Example 6

### Treatment of Gall Bladder Infection

A 67 year old otherwise healthy male subject fell ill after consuming expired, moldy salami meat. Within 3-4 hours after consuming the meat, the subject was admitted to an emergency room at Sherman Oaks Hospital in California. Symptoms included nausea, vomiting every 20 minutes, mild abdominal and severe back pain. Initial diagnosis was biliary colic and hypertension. An electrocardiography test and an ultrasound were administered. The gall bladder was determined to be completely infected, and appeared as an opaque dark mass in the ultrasound, with no visible gallstones (Figs. 5A-5F). The subject was instructed to set an appointment with a general surgeon to have the gall bladder removed. Prescribed were Bentyl 20mg; Norco 10-325 mg; Lisinopril 40 mg. The subject refused to fill these prescriptions, instead opting for treatment with a standard solution of water which contained water clusters at a concentration of approximately 150 million water clusters per drop of water, with an average water droplet size of 500 microns in diameter. Dosage administered was 4 drops of standard solution (as described above) per ounce of water ingested per hour, with an average of 3-4 ounces of water per hour ingested. The subject continued vomiting for three hours. The subject fell asleep for the night, and by 9:00 AM the next morning, pain level was reduced by 85%. The treatment dosage was then reduced to 1-2 drops of standard solution per ounce of water, with approximately 8 ounces of water ingested every 4 hours thereafter. One month and one day after initial diagnosis, the subject went to Northridge Diagnostic Imaging Center to obtain a follow-up ultrasound of the previously abnormal gall bladder. Small gallstones and mild wall thickening were observed (Figs. 6A-6G) and determined to be "normal," and no further medical action was required. The infection had completely subsided as a result of treatment.

### Experiments VI

### Example 7

Additional MIC testing was performed according to the protocols for MIC testing set forth above, with bacteria specific adjustments of growth media and antibiotic used. Results are demonstrated in Figs. 7-9.

The various methods and techniques described above provide a number of ways to carry out the invention. Of course, it is to be understood that not necessarily all objectives or advantages described can be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as taught or suggested herein. A variety of alternatives are mentioned herein. It is to be understood that some preferred embodiments specifically include one, another, or several features, while others specifically exclude one, another, or several features, while still others mitigate a particular feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be employed in various combinations by one of ordinary skill in this art to perform methods in accordance with the principles described herein. Among the various elements, features, and steps some will be specifically included and others specifically excluded in diverse embodiments.

Although the application has been disclosed in the context of certain embodiments and examples, it will be understood by those skilled in the art that the embodiments of the application extend beyond the specifically disclosed embodiments to other alternative embodiments and/or uses and modifications and equivalents thereof.

In some embodiments, the terms "a" and "an" and "the" and similar references used in the context of describing a particular embodiment of the application (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (for example, "such as") provided with respect to certain embodiments herein is intended merely to better illuminate the application and does not pose a limitation on the scope of the application otherwise claimed. No language in the specification should be construed as indicating any non-claimed element essential to the practice of the application.

## Claims

1. A composition comprising water clusters for use in the treatment of a bacterial infection in a subject, wherein the water clusters are present at a concentration of at least 150 million water clusters per drop of water with a water droplet size of 500 microns in diameter.

2. The composition for the use of claim 1, wherein the bacterial infection is a bacterial infection caused by a Gram-negative bacterium.

3. The composition for the use of claim 1, wherein the bacterial infection is a bacterial infection caused by a Gram-positive bacterium.

4. The composition for the use of claim 1, wherein the bacterial infection is a bacterial infection caused by a bacterium selected from the group consisting of *S. pyogenes, S. Mutans, S. pneumonia, S. aureus, K. pneumoniae* and combinations thereof.

5. The composition for the use of claim 1, wherein the bacterial infection is a bacterial infection on a portion of the subject's body selected from the group consisting of: an interior surface of a subject's body, an external surface of a subject's body, and a mucous membrane of a subject's body.

6. The composition for the use of claim 1, wherein the subject is a human.

7. The composition for the use of any of claims 1-6 wherein the composition is administered in a form selected from the group consisting of a powder, a liquid, a solid, a nebulized particle, an atomized particle, and combinations thereof.

## Patentansprüche

1. Zusammensetzung, die Wassercluster umfasst, für die Verwendung bei der Behandlung einer bakteriellen Infektion bei einem Subjekt, wobei die Wassercluster in einer Konzentration von mindestens 150 Millionen Wassercluster pro Tropfen Wasser, mit einer Wassertröpfchengröße von 500 Mikron im Durchmesser, vorliegen.

2. Zusammensetzung für die Verwendung nach Anspruch 1, wobei die bakterielle Infektion eine durch ein gramnegatives Bakterium verursachte bakterielle Infektion ist.

3. Zusammensetzung für die Verwendung nach Anspruch 1, wobei die bakterielle Infektion eine durch ein grampositives Bakterium verursachte bakterielle Infektion ist.

4. Zusammensetzung für die Verwendung nach Anspruch 1, wobei die bakterielle Infektion eine durch ein Bakterium verursachte bakterielle Infektion ist, das aus der Gruppe ausgewählt ist, die aus *S. pyogenes, S. mutans, S. pneumoniae, S. aureus, K. pneumoniae* und Kombinationen davon besteht.

5. Zusammensetzung für die Verwendung nach Anspruch 1, wobei die bakterielle Infektion eine bakterielle Infektion auf einem Teil des Körpers des Subjekts ist, der aus der Gruppe ausgewählt ist, die aus Folgenden besteht: einer inneren Oberfläche des Körpers eines Subjekts, einer äußeren Oberfläche des Körpers eines Subjekts und einer Schleimhaut des Körpers eines Subjekts.

6. Zusammensetzung für die Verwendung nach Anspruch 1, wobei das Subjekt ein Mensch ist.

7. Zusammensetzung für die Verwendung nach einem der Ansprüche 1-6, wobei die Zusammensetzung in einer Form verabreicht wird, die aus der Gruppe ausgewählt ist, die aus einem Pulver, einer Flüssigkeit, einem Feststoff, einem vernebelten Partikel, einem zerstäubten Partikel und Kombinationen davon besteht.

## Revendications

1. Composition comprenant des clusters d'eau pour une utilisation dans le traitement d'une infection bactérienne chez un sujet, dans laquelle les clusters d'eau sont présents selon une concentration d'au moins 150 millions de clusters d'eau par goutte d'eau ayant une taille de gouttelette d'eau d'un diamètre de 500 microns.

2. Composition pour une utilisation selon la revendication 1, l'infection bactérienne étant une infection bactérienne provoquée par une bactérie à Gram négatif.

3. Composition pour une utilisation selon la revendication 1, l'infection bactérienne étant une infection bactérienne provoquée par une bactérie à Gram positif.

4. Composition pour une utilisation selon la revendication 1, l'infection bactérienne étant une infection bactérienne provoquée par une bactérie choisie dans le groupe constitué par *S. pyogenes, S. mutans, S. pneumoniae, S. aureus, K. pneumoniae,* et des combinaisons de celles-ci.

5. Composition pour une utilisation selon la revendication 1, l'infection bactérienne étant une infection bactérienne sur une portion du corps du sujet choisie dans le groupe constitué par : une surface interne du corps d'un sujet, une surface externe du corps d'un sujet, et une muqueuse du corps d'un sujet.

6. Composition pour une utilisation selon la revendication 1, le sujet étant un être humain.

7. Composition pour une utilisation selon l'une quelconque des revendications 1-6, la composition étant administrée sous une forme choisie dans le groupe constitué par une poudre, un liquide, un solide, une particule nébulisée, une particule atomisée, et des combinaisons de celles-ci.
